# EUROPEAN PATENT APPLICATION

(11) **EP 4 183 429 A1**
(43) Date of publication of application: **24.05.2023**
(21) Application number: 21842210.3
(22) Date of filing: 14.07.2021
(51) Int. Cl.: A61L 101/02, B01J 35/02, C01G 39/00, A61L 2/23, C04B 35/495, C04B 35/50

(54) **COMPLEX OXIDE CERAMIC, FUNCTIONAL MATERIAL, AND ARTICLE**

(30) Priority: 15.07.2020 JP 2020121420
(71) Applicant: TOKYO INSTITUTE OF TECHNOLOGY, Tokyo 152-8550 (JP); KANAGAWA INSTITUTE OF INDUSTRIAL SCIENCE AND TECHNOLOGY, Ebina-shi Kanagawa 243-0435 (JP); NGK Spark Plug Co., Ltd., Nagoya-shi, Aichi 461-0005 (JP)
(72) Inventor: NAKAJIMA Akira, Tokyo 152-8550 (JP); ITO Takuro, Tokyo 152-8550 (JP); MATSUSHITA Sachiko, Tokyo 152-8550 (JP); ISOBE Toshihiro, Tokyo 152-8550 (JP); SUNADA Kayano, Ebina-shi, Kanagawa 243-0435 (JP)
(74) Representative: J A Kemp LLP
(86) International application number: PCT/JP2021/026416
(87) International publication number: WO 2022/014631

(57) **Abstract**

Provided are a complex oxide ceramic having high antiviral activity, a functional material, and an article provided with the complex oxide ceramic and/or the functional material. The complex oxide ceramic according to one aspect of the present invention is a complex oxide ceramic containing cerium and molybdenum and having antiviral activity. The functional material according to one aspect of the present invention is also a functional material including the complex oxide ceramic mixed with a photocatalyst and/or an antibacterial effect. The article according to one aspect of the present invention is an article having the complex oxide ceramic and/or the functional material on at least a part of the surface thereof.

## Description

### Technical Field

The present invention relates to a complex oxide ceramic, a functional material, and an article provided with the complex oxide ceramic and/or the functional material.

### Background Art

The global spread of novel coronavirus (COVID-19) has raised the threat of global outbreaks of various viruses (viral pandemics). Once a viral pandemic occurs, it takes at least several months for vaccines to be available in medical institutions. Thus, research on the prevention and control of the epidemic is an urgent issue.

Inorganic antiviral materials have a shorter history than organic ones. However, inorganic antiviral materials have been actively studied in recent years because of their characteristics such as effectiveness against various viruses, applicability in a relatively wide temperate range, and difficulty for viruses to acquire resistance. Antiviral activity has been reported in, for example, metal systems such as Ag and Cu, photocatalytic systems such as TiO₂, ZnO, and CaO, and used in practice. Patent Literature 1 discloses a technique related to a complex oxide ceramic having both self-water repellency and antibacterial and antiviral performance.

### Citation List

### Patent Literature

Patent Literature 1: International Patent Publication No. WO 2020/017493

### Summary of Invention

### Technical Problem

Meanwhile, conventional inorganic antiviral materials have problems such as coloration and reduced activity due to oxidation or the like, and limitations in a use environment (need for light and alkalization). Therefore, it is desired to develop a new inorganic antiviral material having high antiviral activity that can solve these problems. An object of the present invention is thus to provide a complex oxide ceramic having high antiviral activity, a functional material, and an article provided with the complex oxide ceramic and/or the functional material.

### Solution to Problem

A complex oxide ceramic according to one aspect of the present invention is a complex oxide ceramic containing cerium and molybdenum and having antiviral activity.

The complex oxide ceramic may be a complex oxide ceramic represented by Ce₂Mo₃O₁₂.

The complex oxide ceramic may be a complex oxide ceramic represented by Ce₂Mo₄O₁₅.

The complex oxide ceramic may be a complex oxide ceramic represented by Ce₂Mo₃O₁₃.

The complex oxide ceramic may be a complex oxide ceramic represented by Ce(MoO₄)₂, Ce₂MoO₆, Ce₂(MoO₄)₃, Ce₂Mo₃O₁₃, Ce₂Mo₄O₁₅, Ce₆(MoO₄)₈(Mo₂O₇), or Ce₈Mo₁₂O₄₉.

The complex oxide ceramic may be a complex oxide ceramic having a virus reduction rate of 99% or more after 6 hours through the film adhesion method.

The complex oxide ceramic may be a complex oxide ceramic having a virus reduction rate of 99.99% or more after 6 hours through the film adhesion method.

The complex oxide ceramic may be a sintered body.

A functional material according to one aspect of the present invention is a functional material in which the complex oxide ceramic is mixed with a photocatalyst and/or a material having an antibacterial effect.

In the functional material, the material having an antibacterial effect may contain at least one of an oxide containing La and Mo and an oxide containing La, Ce, and Mo.

An article according to one aspect of the present invention is an article having the complex oxide ceramic and/or the functional material on at least a part of the surface thereof.

### Advantageous Effects of Invention

According to the present invention, it is possible to provide a complex oxide ceramic having high antiviral activity, a functional material, and an article provided with the complex oxide ceramic and/or the functional material.

### Brief Description of the Drawings

[Fig. 1] Fig. 1 is a flowchart illustrating an example of a citric acid polymerization method.
[Fig. 2] Fig. 2 is a flowchart illustrating an example of a hydrothermal synthesis method.
[Fig. 3] Fig. 3 is a view showing XRD measurement results of each sample.
[Fig. 4] Fig. 4 is a table showing compositional analysis results (ICP-OES, XPS) of each sample.
[Fig. 5] Fig. 5 is a scanning electron microscope (SEM) photograph of each sample.
[Fig. 6] Fig.6 is a flowchart illustrating an antiviral evaluation procedure.
[Fig. 7] Fig. 7 is a graph showing antiviral evaluation results of each sample.

### Description of Embodiments

The complex oxide ceramic according to the present embodiment is a complex oxide ceramic containing cerium (Ce) and molybdenum (Mo) and having antiviral activity. The composition ratio (ion ratio) of cerium (Ce)to molybdenum (Mo) contained in the complex oxide ceramic is not particularly limited, but may be, for example, Ce:Mo = 2:3 or Ce:Mo = 1:2. The amount of oxygen atoms (O) contained in the complex oxide ceramic may be stoichiometric or may deviate from the stoichiometric composition. In other words, the complex oxide ceramic may be a nonstoichiometric compound.

The complex oxide ceramic according to the present embodiment is preferably a complex oxide ceramic represented by Ce(MoO₄)₂, Ce₂MoO₆, Ce₂(MoO₄)₃, Ce₂Mo₃O₁₃, Ce₂Mo₄O₁₅, Ce₆(MoO₄)₈(Mo₂O₇), or Ce₈Mo₁₂O₄₉. The complex oxide ceramic according to the present embodiment is particularly preferably a complex oxide ceramic represented by Ce₂Mo₃O₁₂, Ce₂Mo₄O₁₅, or Ce₂Mo₃O₁₃.

The complex oxide ceramic of the present embodiment may further contain other elements to the extent that the effect is not impaired. Examples of such other elements include transition metal elements. The transition metal elements may be contained as oxides or in other forms without constituting oxides. In the present embodiment, the content of such other elements is preferably 20% by mass or less, more preferably 10% by mass or less, and still more preferably 5% by mass or less based on the total amount of the complex oxide ceramic containing such other elements.

The complex oxide ceramic of the present embodiment may be monocrystalline or polycrystalline, may be amorphous, for example, glassy, and may be a combination of crystalline and amorphous parts. The crystalline phase may be a single phase or a combination of two or more different phases.

As described above, the complex oxide ceramic according to the present embodiment has antiviral activity. As one example, the complex oxide ceramic of the present embodiment has a virus reduction rate of 99% or more, preferably 99.99% or more after 6 hours through a film adhesion method.

The shape of the complex oxide ceramic of the present embodiment is not particularly limited and may have the desired shape depending on the use. For example, the complex oxide ceramic may be a sintered body that is sintered to the desired shape by a method described later, or the sintered body may be ground into a powder. The complex oxide ceramic of the present embodiment can be used as a powder to have a large surface area per mass, thus efficiently exhibiting antiviral properties.

The present embodiment can provide an article having the complex oxide ceramic on at least a part of the surface thereof. In this article, the surface coated with the complex oxide ceramic has antiviral properties. The article of the present embodiment can be applied to any article for which antiviral properties are required. Examples of such articles include a case of an electronic device such as a personal computer or a smartphone; plumbing equipment such as a bathroom, a washroom, and a kitchen; and articles used for medical supplies such as masks or white coats. In the present embodiment, a part of the surface of the article may be a sintered body of the complex oxide ceramic, or a powder of the complex oxide ceramic may be supported on the surface of the article.

A method of carrying the complex oxide ceramic powder on the surface of an article is appropriately selected according to the article. For example, a complex oxide ceramic film may be formed by spraying a complex oxide ceramic powder onto the surface of an article by an aerosol deposition method. A complex oxide ceramic powder, for example, may be combined with a known binder resin and a solvent and applied as an ink or paste onto the surface of the desired article to form a film containing the complex oxide ceramic. The application method is not particularly limited. Examples of the methods to be employed include application methods such as spray coating, dip coating, and spin coating; and printing methods such as flexography, screen printing, and inkjet printing. A powder of the complex oxide ceramic of the present embodiment may be mixed with a resin, and the resin may be molded to form the desired article. The complex oxide ceramic film may be formed on the surface of an article by using a physical vapor deposition (PVD) method such as sputtering or pulsed laser deposition (PLD). In this case, a sintered body of the complex oxide ceramic can be used as a target.

In addition, a base material such as a resin film, paper, glass, fiber, or metal may be prepared to form a laminate provided with a film containing the complex oxide ceramic according to one embodiment on the base material by the aforementioned method. The laminate may be further bonded to an arbitrary article for use.

For example, a mask, a white coat, or the like having excellent antiviral properties can be produced by applying a complex oxide ceramic powder to fibers of a cloth, a non-woven fabric, or the like by employing the aforementioned method. The complex oxide ceramic of the present embodiment may be mixed with a photocatalyst (such as titanium oxide and titanium apatite) to constitute a functional material.

The complex oxide ceramic of the present embodiment may be also mixed with a material having an antibacterial effect to constitute a functional material. Since the complex oxide ceramic of the present embodiment has excellent antiviral properties, it can be used in combination with a material having an antibacterial effect to form a functional material having both antiviral and antibacterial properties. Examples of the antibacterial material include LMO (oxide containing La and Mo) and LCMO (oxide containing La, Ce, and Mo) but are not limited to these materials. Such a functional material may be provided on at least a part of the surface of an article to constitute an article according to the present embodiment.

The method for producing the complex oxide ceramic of the present embodiment is not particularly limited, and the complex oxide ceramic can be obtained by forming a complex oxide containing cerium (Ce) and molybdenum (Mo) and firing it. Examples of suitable production methods include a citric acid polymerization method and a hydrothermal synthesis method which will be particularly described later, but the complex oxide ceramic may be produced by, for example, a solid phase reaction method which will be described later, or other production methods. Hereinafter, the citric acid polymerization method, hydrothermal synthesis method, and solid phase reaction method will be described.

### <Citric Acid Polymerization Method>

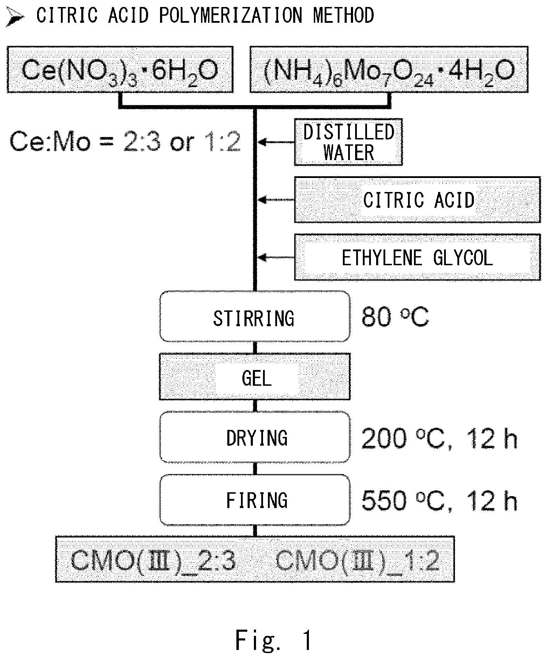

The citric acid polymerization method is described with reference to Fig. 1. Fig. 1 is a flowchart illustrating an example of the citric acid polymerization method.

The citric acid polymerization method of the present embodiment includes:
a gelation step of adding an oxycarboxylic acid and a glycol to an aqueous solution containing a cerium-containing compound and a molybdenum-containing compound, and then heating and stirring the resulting solution to cause an ester reaction between the oxycarboxylic acid and the glycol to form a gel;
a drying step of drying the gel obtained in the gelation step; and
a firing step of firing a powder obtained by drying the gel.

The above-mentioned citric acid polymerization method has advantages of, for example, giving a complex oxide ceramic having excellent uniformity and high density at a relatively low temperature.

In the gelation step, the cerium-containing compound and the molybdenum-containing compound are first mixed with water to form an aqueous solution. An oxycarboxylic acid is then added to the aqueous solution to form a metal oxycarboxylic acid complex. Subsequently, glycol is added thereto to cause an ester reaction between the oxycarboxylic acid and the glycol to form a gel.

Examples of the cerium-containing compound include cerium nitrate. Examples of the molybdenum-containing compound include ammonium molybdate. For example, cerium nitrate hexahydrate (Ce(NO₃)₃·6H₂O) and ammonium molybdate tetrahydrate ((NH₄)₆Mo₇O₂₄·4H₂O) can be used as cerium nitrate and ammonium molybdate, respectively.

Examples of the oxycarboxylic acid include citric acid, and examples of the glycol include ethylene glycol and propylene glycol.

The obtained gel is dried sufficiently in the drying step. The drying method is not particularly limited, but heat drying (for example, at 200°C for 12 hours) is preferred. Then, the powder obtained by drying the gel is fired (firing step). The firing conditions are not particularly limited, but for example, firing can be performed at 550°C for 12 hours. The atmosphere in firing is not particularly limited, but the firing can be performed, for example, in the air. Note that a calcination step of calcinating the powder obtained by drying the gel and a forming step of forming the powder after the calcination may be added between the drying step and the firing step.

### <Hydrothermal Synthesis Method>

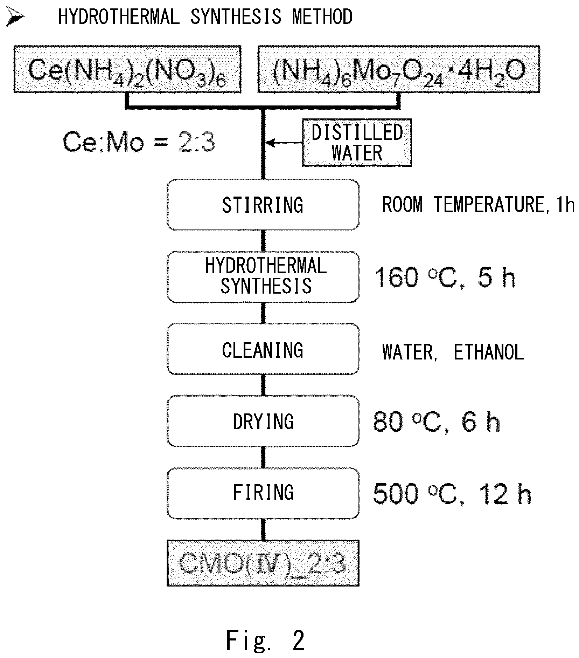

Next, the hydrothermal synthesis method will be described as another method for producing a complex oxide ceramic. Fig. 2 is a flowchart illustrating an example of a hydrothermal synthesis method.

The hydrothermal synthesis method in the present embodiment includes:
a step of stirring and mixing an aqueous solution containing a cerium-containing compound and a molybdenum-containing compound, and then heating and reacting them to give an intermediate material;
a drying step of drying the obtained intermediate material; and
a firing step of firing the powder obtained by drying.

The above-mentioned hydrothermal synthesis method has advantages of, for example, giving a complex oxide ceramic having excellent uniformity and high density at a relatively low temperature.

Specifically, a cerium-containing compound having water solubility first is dissolved in distilled water to prepare a cerium-containing aqueous solution. A molybdenum-containing compound having water solubility is also dissolved in distilled water to prepare a molybdenum-containing aqueous solution. These aqueous solutions are then mixed and stirred at ordinary temperature (room temperature). After that, the mixed aqueous solution is heated for a predetermined time (hydrothermal synthesis) to obtain an intermediate substance.

Examples of the cerium-containing compound having water solubility include cerium nitrate. Examples of the molybdenum-containing compound having water solubility include ammonium molybdate. For example, cerium diammonium nitrate (Ce(NH₄)₂(NO₃)₆) and ammonium molybdate tetrahydrate ((NH₄)₆Mo₇O₂₄·4H₂O) can be used as the cerium-containing compound and the molybdenum-containing compound, respectively.

The obtained intermediate material is cleaned with water and ethanol and then dried sufficiently in the drying step. The drying method is not particularly limited, but heat drying (for example, at 80°C for 6 hours) is preferred. Then, the powder obtained by drying is fired (firing step). The firing conditions are not particularly limited, but for example, firing can be performed at 500°C for 12 hours. The atmosphere in firing is not particularly limited, but the firing can be performed, for example, in the air. Note that a calcination step of calcinating the powder obtained in the drying step and a forming step of forming the powder after the calcination may be added between the drying step and the firing step.

### <Solid Phase Reaction Method>

Next, the solid phase reaction method will be described as an alternative method for producing a complex oxide ceramic. When the solid phase reaction method is employed, powders of a cerium-containing compound and a molybdenum-containing compound are first mixed and calcined to give a calcined powder. For example, a cerium oxide (CeO₂) and a molybdenum oxide (MoOs) can be used as raw materials for the cerium-containing compound and the molybdenum-containing compound, respectively. The conditions for calcination are not particularly limited, but for example, the mixture is preferably calcinated at 900°C or higher. Then, in the firing step, the obtained calcined powder is fired, whereby a sintered body of the complex oxide ceramic can be obtained. The conditions of the firing step are not particularly limited, but for example, firing is preferably performed at 1000°C or higher.

### Examples

Hereinafter, the present embodiment will be specifically described with Examples. These descriptions are not intended to limit the present embodiment.

### <Preparation of Sample>

Samples according to Examples 1 to 3 and Comparative Examples 1 and 2 were prepared using the following method.

### [Example 1: CMO(III)_2:3]

As a sample according to Example 1, a compound represented by Ce₂Mo₃O₁₂ was prepared. The sample according to Example 1 was prepared using the citric acid polymerization method (see Fig. 1). Hereinafter, the method for preparing a sample will be specifically described.

First, cerium (III) nitrate hexahydrate (Ce(NO₃)₃·6H₂O) and ammonium molybdate (VI) tetrahydrate ((NH₄)₆Mo₇O₂₄·4H₂O) were each dissolved in distilled water. Once a homogeneous solution was obtained, ammonium molybdate (VI) solution was slowly added dropwise to the cerium (III) nitrate solution with stirring so that an ion ratio of cerium to molybdenum was Ce:Mo = 2:3.

Thereafter, citric acid was dissolved in and added to distilled water in an amount of substance twice that of the metal ions (Ce + Mo) in the mixed solution. Next, an esterification reaction was induced by adding ethylene glycol in an amount of 2/3 substance of citric acid. This solution was stirred at 80°C for several hours to evaporate the water, thereby obtaining a gel. The obtained gel was dried at 200°C for 12 hours, then fired at 550°C for 12 hours, and ground to give a specimen powder.

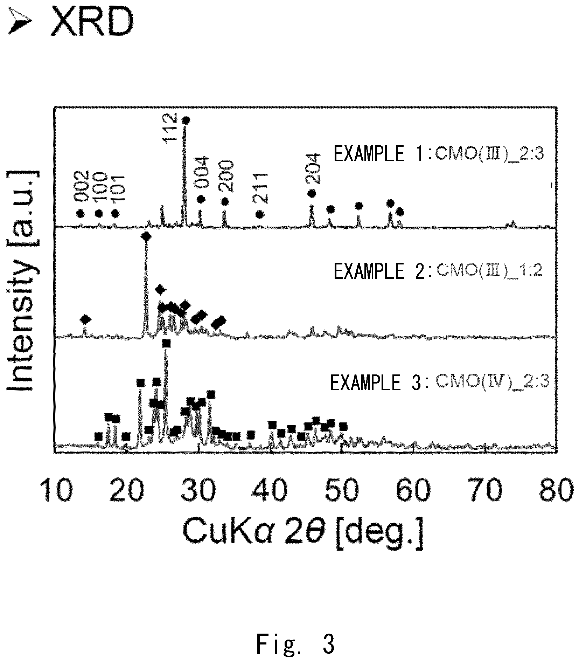

After that, the obtained specimen powder was analyzed using X-ray diffraction and found to be a single phase powder of Ce₂Mo₃O₁₂ (see Fig. 3). Hereafter, the sample according to Example 1 will also be referred to as CMO(III)_2:3.

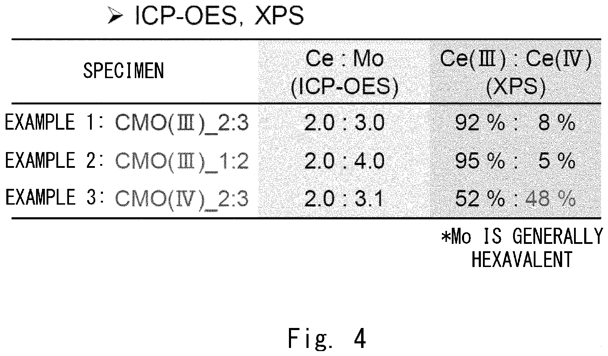

The composition of the obtained specimen powder (CMO(III)_2:3) was analyzed using an inductivity coupled plasma optical emission spectrometer (ICP-OES), and it was found that Ce: Mo = 2.0:3.0 (see Fig. 4). Therefore, a sample with the planned composition ratio (Ce:Mo = 2:3) could be prepared. In addition, the valence of Ce atoms on the powder surface was investigated using XPS (X-ray photoelectron spectroscopy), and it was found that Ce(III):Ce(IV) = 92%:8% (see Fig. 4). Note that Mo was generally hexavalent.

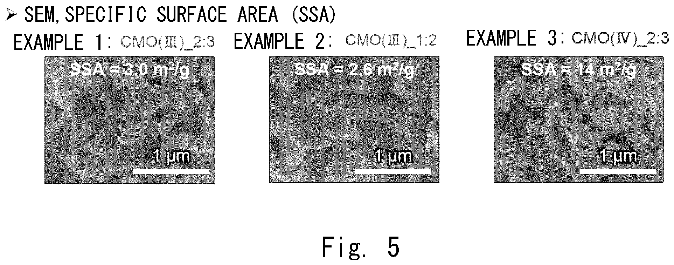

Besides, Fig. 5 shows scanning electron microscope (SEM) photographs of the obtained specimen powder. The specific surface area values obtained from nitrogen adsorption and BET methods are also shown. In CMO(III)_2:3 according to Example 1, the specific surface area value was SSA = 3.0 (m²/g).

### [Example 2:CMO(III)_1:2]

As a sample according to Example 2, a compound represented by Ce₂Mo₄O₁₅ was prepared. The sample according to Example 2 was prepared using the citric acid polymerization method (see Fig. 1). Hereinafter, the method for preparing a sample will be specifically described.

First, cerium (III) nitrate hexahydrate (Ce(NO₃)₃·6H₂O) and ammonium molybdate (VI) tetrahydrate ((NH₄)₆Mo₇O₂₄·4H₂O) were each dissolved in distilled water. Once a homogeneous solution was obtained, the ammonium molybdate (VI) solution was slowly added dropwise to the cerium (III) nitrate solution with stirring so that an ion ratio of cerium to molybdenum was Ce:Mo = 1:2.

Thereafter, citric acid was dissolved in and added to distilled water in an amount of substance twice that of the metal ions (Ce + Mo) in the mixed solution. Next, an esterification reaction was induced by adding ethylene glycol in an amount of 2/3 substance of citric acid. This solution was stirred at 80°C for several hours to evaporate the water, thereby obtaining a gel. The obtained gel was dried at 200°C for 12 hours, then fired at 550°C for 12 hours, and ground to give a specimen powder.

After that, the obtained specimen powder was analyzed using X-ray diffraction and found to be a single phase powder of Ce₂Mo₄O₁₅ (see Fig. 3). Hereafter, the sample according to Example 2 will also be referred to as CMO(III)_1:2.

The composition of the obtained specimen powder (CMO(III)_1:2) was analyzed using an inductivity coupled plasma optical emission spectrometer (ICP-OES), and it was found that Ce:Mo = 2.0:4.0 (see Fig. 4). Therefore, a sample with the planned composition ratio (Ce:Mo = 2:4) could be prepared. In addition, the valence of Ce atoms on the powder surface was investigated using XPS, and it was found that Ce(III):Ce(IV) = 95%:5% (see Fig. 4). Note that Mo is generally hexavalent.

Besides, Fig. 5 shows scanning electron microscope (SEM) photographs of the obtained specimen powder. The specific surface area values obtained from nitrogen adsorption and BET methods are also shown. In CMO(III)_1:2 according to Example 2, the specific surface area value was SSA = 2.6 (m²/g).

### [Example 3: CMO(IV)_2:3]

As a sample according to Example 3, a compound represented by Ce₂Mo₃O₁₃ was prepared. The sample according to Example 1 was prepared using the hydrothermal synthesis method (see Fig. 2). Hereinafter, the method for preparing a sample will be specifically described.

First, cerium diammonium nitrate (Ce(NH₄)₂(NO₃)₆) and ammonium molybdate (VI) tetrahydrate ((NH₄)₆Mo₇O₂₄·4H₂O) were separately dissolved in distilled water. Once homogeneous solutions were obtained, the ammonium molybdate (VI) solution was slowly added dropwise to the cerium diammonium nitrate solution that is stirred, followed by stirring for 1 hour, and the resulting solution was poured into a teflon container. An ion ratio of cerium to molybdenum was Ce:Mo = 2:3.

Thereafter, the hydrothermal synthesis was carried out at 160°C for 5 hours. Subsequently, the powder obtained by the hydrothermal synthesis was cleaned with water and ethanol and dried at 80°C for 6 hours to give a dry powder. The dry powder was then fired at 500°C for 12 hours to give a specimen powder.

After that, the obtained specimen powder was analyzed using X-ray diffraction and found to be a single phase powder of Ce₂Mo₃O₁₃ (see Fig. 3). Hereafter, the sample according to Example 3 will also be referred to as CMO(IV)_2:3.

The composition of the obtained specimen powder (CMO(IV)_2:3) was analyzed using an inductivity coupled plasma optical emission spectrometer (ICP-OES), and it was found that Ce:Mo = 2.0:3.1 (see Fig. 4). Therefore, a sample with the composition ratio (Ce:Mo = 2:3) approximately as planned could be prepared. In addition, the valence of Ce atoms on the powder surface was investigated using XPS, and it was found that Ce(III):Ce(IV) = 52%:48% (see Fig. 4). Note that Mo is generally hexavalent.

Besides, Fig. 5 shows scanning electron microscope (SEM) photographs of the obtained specimen powder. The specific surface area values obtained from nitrogen adsorption and BET methods are also shown. In CMO(IV)_2:3 according to Example 3, the specific surface area value was SSA = 14 (m²/g).

### [Comparative Example: LCMO, LMO]

La_{1.8}Ce_{0.2}Mo₂O₉ (LCMO) and La₂Mo₂O₉(LMO) were each prepared as a sample according to Comparative Example.

In preparing LCMO, first, a solution of lanthanum nitrate hexahydrate (La(NO₃)₃·6H₂O) and cerium nitrate hexahydrate (Ce(NO₃)₃.6H₂O) dissolved in distilled water and a solution of ammonium molybdate tetrahydrate ((NH₄)₆Mo₇O₂₄.4H₂O) dissolved in distilled water were each prepared. These aqueous solutions were then mixed at room temperature. Next, the mixture was kept in a thermostatic bath at 70°C for 24 hours to give an intermediate substance.

Subsequently, the intermediate substance was dried at 120°C for 24 hours to give a dry powder. Then, the powder was subjected to calcination by keeping it at 500°C for 6 hours under an air atmosphere to give a calcined powder of the complex oxide ceramic (La_{1.8}Ce_{0.2}Mo₂O₉(LCMO)). Thereafter, the obtained calcined powder was fired at 900°C for 3 hours in the air to give a sintered body of LCMO.

In preparing LMO, an aqueous solution of lanthanum nitrate hexahydrate (La (N0₃)₃• 6H₂O) and an aqueous solution of ammonium molybdate tetrahydrate ((NH₄)₆Mo₇O₂₄·4H₂O) were first prepared. The two aqueous solutions were mixed so that the molar ratio of La to Mo was 1: 1. After the solution was warmed to 80°C, an aqueous citric acid solution was added thereto so that the molar ratio of citric acid to the sum of La and Mo was 1:2. Next, an ethylene glycol solution was added so that the amount of ethylene glycol relative to citric acid was 2/3 equivalents, and the solution was kept in a thermostatic bath at 80°C for 6 hours with stirring to give a gel.

Subsequently, the gel was dried at 200°C for 24 hours to give a dry powder. Then, the powder was subjected to calcination by keeping it at 500°C for 12 hours under an air atmosphere to give a calcined powder of the complex oxide ceramic (La₂Mo₂O₉(LMO)). Thereafter, the obtained calcined powder was fired at 900°C for 12 hours in the air to give a sintered body of LMO.

### <Antiviral Evaluation>

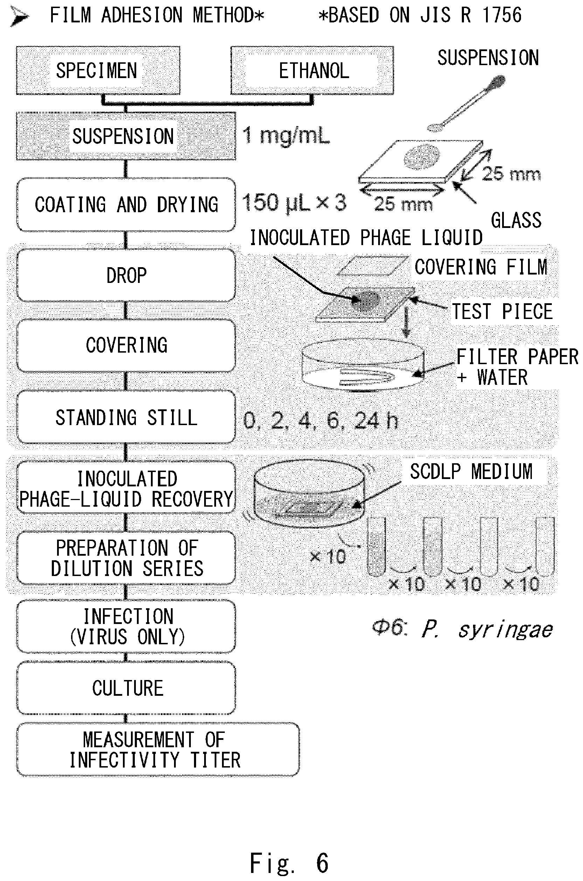

Antiviral evaluation will be described with reference to Fig. 6. Fig. 6 is a flowchart illustrating an antiviral evaluation procedure. Fig. 6 also shows a schematic diagram illustrating an antiviral evaluation method.

The samples prepared by the above-mentioned production methods were dispersed in ethanol to prepare a 1 mg/ml dispersion. 150 µL of the dispersion was applied onto a 25-mm square glass substrate and dried. The application and drying were repeated three times, followed by sterilization to prepare a plurality of test substrates.

Separately, each bacteriophage ϕ6 (substitute for influenza virus) was dissolved in 1/500 NB medium to make an inoculated phage liquid of about 2.0 × 10⁹ PFU/ml, which was then diluted 100-fold to yield a solution of about 2.0 × 10⁷ PFU/ml.

Each of the test substrates was inoculated with 50 µL of the inoculated phage liquid (approximately 10⁶ PFU), covered tightly with a film, wrapped in aluminum foil, and allowed to stand still in the dark. To count the number of remaining phages (plaque count) among the samples after a predetermined time, the growth of the virus count was suppressed in SCDLP medium and then diluted with PBS. Subsequently, a solution of ϕ6 infected with *Pseudomonas syringae* (*P. syringae*) that had been mixed with soft agar medium was added to dishes seeded with LB agar medium and allowed to stand for a predetermined time. After that, the number of plaques was recorded to determine the antiviral activity value.

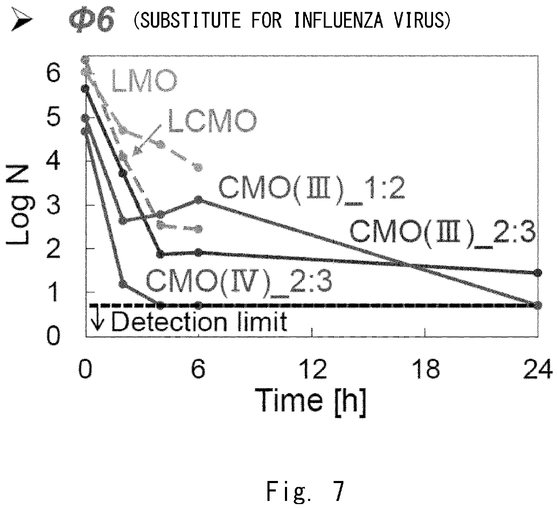

Fig. 7 shows antiviral test results. In Fig. 7, the vertical axis represents the logarithm of the titer of the virus (bacteriophage ϕ6), and a decrease of two digits indicates antiviral activity. As shown in Fig. 7, CMO(III)_2:3 according to Example 1, CMO(III)_1:2 according to Example 2, and CMO(IV)_2:3 according to Example 3 exhibited better antiviral activity than LCMO and LMO according to Comparative Examples. In particular, CMO(IV)_2:3 according to Example 3 exhibited the best antiviral activity against bacteriophage ϕ6. Specifically, CMO(IV)_2:3 according to Example 3 exhibited antiviral activity sufficient to kill 99.99% or more of bacteriophage ϕ6 in 6 hours.

While the present invention has been described based on the embodiments above, the present invention is not limited to the arrangements of the embodiments. Various modifications, changes, and combinations as would be obvious to those skilled in the art may be made within the scope of the claims of the present invention.

This application claims a priority based on Japanese Patent Application No. 2020-121420 filed on July 15, 2020, the disclosure of which is incorporated herein in its entirety by reference.

## Claims

1. A complex oxide ceramic having antiviral activity, comprising cerium and molybdenum.

2. The complex oxide ceramic according to Claim 1, wherein the complex oxide ceramic is represented by Ce₂Mo₃O₁₂.

3. The complex oxide ceramic according to Claim 1, wherein the complex oxide ceramic is represented by Ce₂Mo₄O₁₅.

4. The complex oxide ceramic according to Claim 1, wherein the complex oxide ceramic is represented by Ce₂Mo₃O₁₃.

5. The complex oxide ceramic according to Claim 1, wherein the complex oxide ceramic is represented by Ce(MoO₄)₂, Ce₂MoO₆, Ce₂(MoO₄)₃, Ce₂Mo₃O₁₃, Ce₂Mo₄O₁₅, Ce₆(MoO₄)₈(Mo₂O₇), or Ce_{S}Mo₁₂O₄₉.

6. The complex oxide ceramic according to any one of Claims 1 to 5,
wherein the complex oxide ceramic has a virus reduction rate of 99% or more after 6 hours through a film adhesion method.

7. The complex oxide ceramic according to any one of Claims 1 to 5,
wherein the complex oxide ceramic has a virus reduction rate of 99.99% or more after 6 hours through a film adhesion method.

8. The complex oxide ceramic according to any one of Claims 1 to 7,
wherein the complex oxide ceramic is a sintered body.

9. A functional material, wherein the complex oxide ceramic according to any one of Claims 1 to 8 is mixed with a photocatalyst and/or a material having an antibacterial effect.

10. The functional material according to Claim 9, wherein the material having an antibacterial effect comprises at least one of an oxide containing La and Mo and an oxide containing La, Ce, and Mo.

11. An article having the complex oxide ceramic and/or the functional material according to any one of Claims 1 to 10 on at least a part of a surface thereof.
